# Europäisches Patentamt

# European Patent Office

# Office européen des brevets

(11) Publication number: **0 153 327**
**B1**

(12) **EUROPEAN PATENT SPECIFICATION**

(45) Date of publication of patent specification: **23.05.90**

(51) Int. Cl.⁵: **A 61 L 2/10, A 61 M 31/00**

(21) Application number: **84902638.0**

(22) Date of filing: **15.06.84**

(86) International application number:
**PCT/US84/00929**

(87) International publication number:
**WO 85/00979 14.03.85 Gazette 85/07**

(54) **DEVICE FOR PROVIDING ANTIBACTERIAL RADIATION.**

(30) Priority: **19.08.83 US 524704**
**03.11.83 US 548389**

(43) Date of publication of application:
**04.09.85 Bulletin 85/36**

(45) Publication of the grant of the patent:
**23.05.90 Bulletin 90/21**

(84) Designated Contracting States:
**BE CH DE FR GB LI NL SE**

(56) References cited:
**WO-A-82/04188**
**US-A-3 986 508**
**US-A-4 196 730**
**US-A-4 433 244**
**US-A-4 439 193**

**N, Nephrology Nurse, March/April 1981, Alex E.
Zacharias, R.N., CAPD for the Blind, pages 53-54**

(73) Proprietor: **BAXTER INTERNATIONAL INC. (a
Delaware corporation)**
**One Baxter Parkway**
**Deerfield Illinois 60015 (US)**

(72) Inventor: **KULIN, Daniel**
**1700 Busse Road**
**Marengo, IL 60052 (US)**
Inventor: **GRANZOW, Daniel, B.**
**33666 N. Oak Drive**
**Ingleside, IL 60041 (US)**
Inventor: **DANIELSEN, Brant, R.**
**1009 N. Clarendon Drive**
**Round Lake Beach, IL 60073 (US)**
Inventor: **MUNSCH, John, M.**
**912 Wexford Court**
**Libertyville, IL 60048 (US)**
Inventor: **BELLOTTI, Marc**
**1215 Willow Road**
**Winnetka, IL 60093 (US)**
Inventor: **TAYLOR, Larry, C.**
**5421 Shore Drive**
**McHenry, IL 60050 (US)**

(74) Representative: **MacGregor, Gordon et al
ERIC POTTER & CLARKSON 14 Oxford Street
Nottingham, NG1 5BP (GB)**

Courier Press, Leamington Spa, England.

## Description

Technical Field and Prior Art

In various fields of medicine and elsewhere, and particularly in continuous ambulatory peritoneal dialysis (CAPD) there is a need to make and break connnections between peritoneal tubing communicating with the peritoneal cavity and a source of peritoneal dialysis solution with substantially sterile procedure. At the same time it is desirable for patients undergoing CAPD or another form of peritoneal dialysis on a chronic basis to be released from close supervision by medical personnel and permitted to do the dialysis solution exchange procedures independently at their homes or places of work. However, the need for maintenance of substantially sterile procedure remains critical if peritonitis is to be avoided, particularly in the case of patients on CAPD.

In response to this, various systems for irradiation of connectors have been proposed, and one has been commercially developed, in which at least the outer connector is made of ultraviolet transparent material, and after the connectors are brought together but before seals are released to permit flow of solution through the newly formed connection, the connectors are irradiated with ultraviolet light for antibacterial effect outside and inside the connectors. See WO—A—82/04188; Kulin et al. US—A—4,412,834, entitled "Antimicrobial Ultraviolet Irradiation of Connector for Continuous Ambulatory Peritoneal Dialysis"; and Hogan US—A—4,433,244, entitled "Apparatus for Irradiating Tubing Connectors". In all of the above references, ultraviolet radiation is used for antibacterial effect in the newly formed connection.

Patients who are undergoing pertioneal dialysis on a chronic basis are often elderly, ill, and debilitated. The disease which requires life maintenance by dialysis interferes with nerve conductivity which, in turn, reduces their manual dexterity. Accordingly, it becomes desirable in the case of many patients to provide them with all assistance possible in the making and breaking of connections during peritoneal dialysis and particularly CAPD operations.

In response to this, automatic systems for making and breaking connections have been provided. For example, the Steri-Track device which has been used and which is described in an article entitled "CAPD For the Blind" from the periodical *Nephrology Nurse,* March/April, 1981, pp. 53—54. This device is a self-contained portable device. When doing bag exchanges, a fresh bag of dialyzate is placed into a stationary end of a holder. At this point the protective tab of the bag is removed, and a spike is taken from the discharging bag and fitted into the grooves of a sliding plate. The patient now manipulates the sliding plate toward the bag with the result that the spike will plunge into the port of the bag with alleged 98 percent probabilty.

In accordance with the preferred embodiment of this invention, an automatic connection and disconnection system is provided in conjunction with a radiation system for exerting antibacterial effect on connectors before or after joining, in a peritoneal dialysis system or other system as may be desired. A variable mechanical advantage advancement system of the first and second means that carry the respective connectors is provided so that, preferably, even a debilitated person can exert the amount of force necessary to make and break the connection between the connectors, while at the same time the connectors can be separated by a desired substantial distance with a simple short motion of the handle.

This and other advantages and features are more fully described in the description below.

The present invention provides radiation sterilization apparatus for a pair of connectors to be joined together, the connectors being carried at the ends of flow conduits, and said apparatus comprising a housing means within said housing for providing antibacterial radiation, and first and second means for holding the connectors within the housing in a position to be irradiated by said radition, characterised by drawer means carrying said first and second means to permit exposure of said connectors to antibacterial radiation when said drawer means is closed and to permit manual access to said connectors when mounted in said first and second means when the drawer is opened, at least one of said first and second means being movable between first and second positions, by control means operable from outside said housing to cause connectors carried in the first and second means to be separated from each other in the first position, and to cause said carried connectors to be joined together in the second position.

The pre-characterising clause of Claim 1 is based on WO—A—82/04188.

Rack and gear means can be provided for relatively moving the first and second means between the first and second positions. The gear of the rack and gear means defines gear teeth of increasing pitch, i.e., distance of the center point of each gear tooth from the gear's center of rotation. This increasing pitch relationship is defined about at least a portion of the periphery of the gear. The rack of the rack and gear means defines mating teeth correspondingly spaced along its length in positions to permit relatively linear motion betwen the rack and gear as the gear rolls along the rack. As the result of this, the mechanical advantage of the rack and gear means varies with the position of the gear along the rack because of the varying pitch of the gear teeth. Preferably, the mechanical advantage is substantially at a maximum when the rack and gear means are at the second position.

In other words, the gear teeth which engage the teeth of the rack at the second position are of minimum pitch, i.e., their distance to the center of rotation is minimum, resulting in the highest mechanical advantage in the system. Accordingly, it becomes easier for anybody including debilitated patients to make or break a tight, frictional connection or disconnection, assisted by the increased mechanical advantage of the rack and gear means at this second position, which corresponds to the connected position of the connectors.

However, as the connectors are pulled apart, the mechanical advantage decreases as the gear moves along the rack, with teeth of increasing pitch being in contact with the rack. Accordingly, after the original, tight, frictional connection between the connectors is broken, the mechanical advantage is reduced, causing the rack to move faster as the gear rotates at a constant rate.

Thus, the simple operation of the handle to disconnect the connectors results in an initial, high mechanical advantage for breaking the connection, and then a reduced mechancial advantage to cause the connectors to thereafter move apart more rapidly for less required motion of the handle.

It is generally preferred for the handle attached to the gear to be rotatable only about 180° which, because of the variable mechanical advantage system of this invention, is sufficient to permit the first and second means to be spaced far enough apart to sufficiently space the connectors carried in the first and second means in the first position. Thus connectors can be respectively placed in the first and second means, and the handle turned to actuate the rack and gear means, causing the connectors to be driven toward each other initially rapidly, and then more slowly, with higher mechanical advantage, so that a debilitated person does not have to struggle with the final, frictional locking of the connectors in to their connected relationship. Similarly, breaking of the frictional relationship between the connectors is also easy in this system.

The teeth of the rack may be positioned at differing distances from an axis of relative motion, i.e., any axis defining the relative motion between the rack and gear between the first and second positions. These differing distances of the gear teeth of the rack may correspond inversely to the differing pitches of the gear teeth, to permit relatively linear motion along the axis. Specifically, the sum of the pitch of each gear tooth and the distance from said axis of a rack tooth meshing against such gear tooth may be substantially constant from gear tooth to gear tooth along the entire length of the rack and gear system as the gear rolls along the rack. The result of this is to permit relatively linear motion of the rack along an axis between the first and second positions, even though the pitches of the gear teeth are variable.

The housing may also define latch means and a latch engaging site, for example an engaging hook and wall, described in greater detail below, which prevents reopening of the drawer means after initial closing until the second means is moved to the second position.

Description of the Drawings

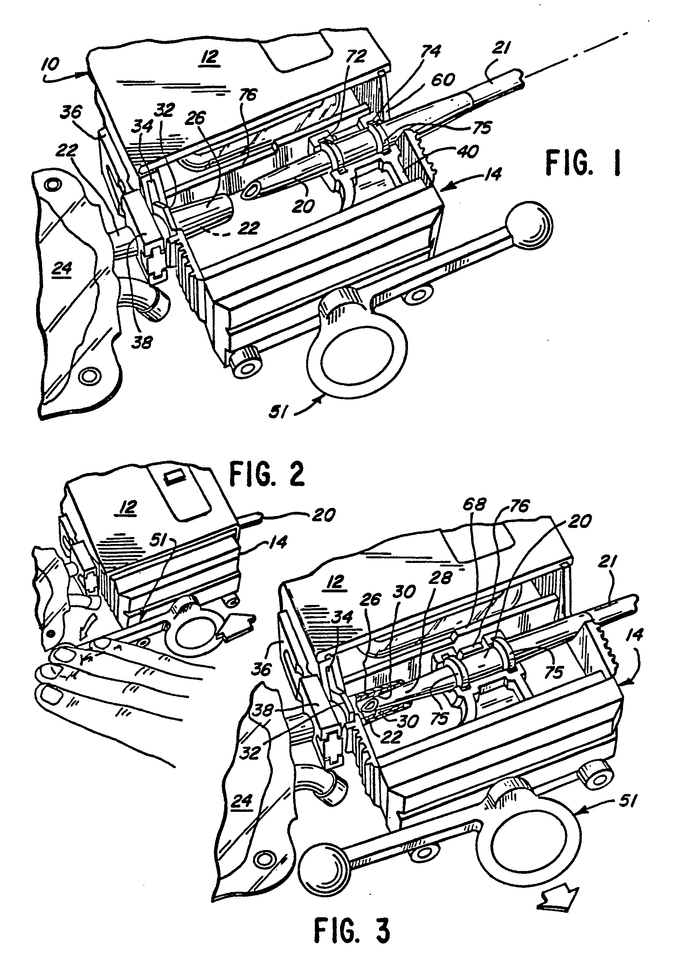

In the drawings, Figure 1 is a fragmentary, perspective view of apparatus of this invention, shown to be holding a pair of connectors for use in peritoneal dialysis.

Figure 2 is a fragmentary, perspective view of the apparatus of Figure 1, showing it in an intermediate configuration of use.

Figure 3 is a fragmentary, perspective view similar to Figure 1, showing the apparatus of this invention after the connection between the connectors has been made and the antibacterial radiation process has been applied.

Figures 4 and 5 are fragmentary perspective views of the second means and the rack and gear means of this invention, shown in different positions.

Figure 6 is a fragmentary, longitudinal sectional view of the apparatus of this invention with the drawer closed.

Figure 7 is a sectional view taken along line 7—7 of Figure 6.

Figure 8 is a fragmentary sectional view taken along line 8—8 of Figure 7.

Figure 9 is a fragmentary transverse sectional view of the apparatus of this invention taken along line 9—9.

Description of Specific Embodiment

Referring to Figures 1—3, the apparatus 10 of this invention includes housing 12 and a slide drawer 14. A convoluted ultraviolet bulb 16 within housing 12 may define a channel 18 (Figure 10) between the respective convolutions of bulb 16 into which the respective connectors 20, 22 may be placed by closing of drawer 14 for ultraviolet irradiation thereof. Connector 20 may connect to peritoneal transfer set tubing 21, while connector 22 may be a flexible, diaphragm-containing tube carried by peritoneal dialysis solution bag 24.

The design of apparatus 10 including housing 12, drawer 14, and ultraviolet bulb 16 may be generally similar to the design disclosed in the cited Hogan patent, except for the design changes described herein. Also, the housing, drawer, and ultraviolet bulb used in this invention may be generally similar in design except as otherwise indicated herein to the germicidal chamber currently available to the public from Travenol Laboratories, Inc. for irradiating connections in CAPD with ultraviolet radiation.

Connector 22 is positioned within a separable, molded member which comprises shaping sleeve 26 proportioned to receive the flexible end of connector 22 and to shape it as shown in Figure 3 into desired, circular configuration, to receive the spike 28 of connector 20, puncturing diaphragm 30 is a conventional manner. The molded member containing sleeve 26 also carries hexagonal flange 32, which can fit in first means 34 for holding connector 22, which is shown to be a generally U-shaped trough, receiving flange 32 and holding the structure in position.

The molded member also includes sliding clamp member 36, positioned in a slide box 38, which may be of the general design of analogus structure shown in Lueders et al. U.S. Application Serial No. 338,712, file January 11, 1982 and entitled "Continuous Amubulatory Peritoneal Dialysis Clamping System", to serve as a slide clamp to control flow through the flexible tubing which comprises connector 22, connected to peritoneal dialysis solution bag 24.

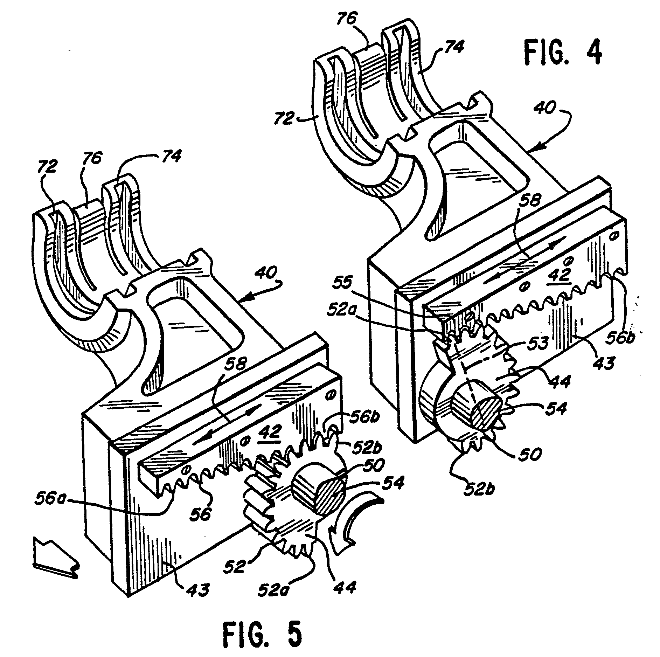

Second connector 20 is carried by second means 40 for holding the connector. Second means 40, as shown by Figures 4—7, is carried as part of a rack and gear system including rack 42 and gear 44. Rack 42 is attached to and can move with plate 43, while gear 44 is in a fixed position. Second means 40 is shown to be attached to rack 42 and to slide in a track groove 46 defined by the front panel 48 of drawer 14.

Gear 44 rotates about shaft 50, which terminates in handle 51. As handle 51 is rotated, gear 44 corrspondingly rotates, to move rack 42 between the respective first and second positions illustrated respectively in Figures 1 and 4, and Figures 3 and 5. Rack and gear system 42, 44 can be purchased from Cunningham Industries of Stamford, Connecticut.

It can be seen that teeth 52 of gear 44 define increasing pitch (distance) from the centre of each gear tooth 52 to the center of axis rotation 54, extending approximately 180° about gear 44, so that the pitch or distance of gear tooth 52a from center of rotation 54 is much greater than the corresponding pitch or distance of gear tooth 52b.

It can also be seen that teeth 56 of rack 42 are correspondingly not in parallel array with axis of rack movement 58 between the first and second positions, but are positioned at differing distances from axis 58 so that rack tooth 56a is closer to axis 58 than rack tooth 56b. The spacing from axis 58 of the respective teeth 56 corresponds inversely to the differing pitches of gear teeth 52 so that rack 42 can move in relatively linear motion along axis 58. Specifically, the sum of the pitch of each gear tooth 52 and the distance from axis 58 of a rack tooth 56 meshing against such gear tooth is substantially constant. In other words, the sum of the pitch 53 of gear tooth 52a from axis of rotation 54 and the perpendicular distance 55 of rack tooth 56a from axis 58 (Figure 4) should be substantially equal to the corresponding pitch of gear tooth 52b plus the distance from axis 58 of gear tooth 56b. Thus as the pitch of each gear tooth increases, the perpendicular distance of each rack tooth from axis 58 correspondingly decreases. This permits substantially linear, perpendicular motion of rack 42 relative to axis of rotation 54.

Drawer 14 is conventionally slidable into and out of housing 12, taking first means 34 and second means 40 for holding the respective connectors with it. As shown, first means 34, the U-shaped trough, is open at the top and defined in a side wall of drawer 14. On the opposite side wall of drawer 14 an open slot 60 is provided to receive connector 20 of tubing 21 in sliding relation to it. Housing 12 carries horizontal slots to intersect the slots defined by first means 34 and slot 60, to permit closing of drawer 14 with the respective connectors in place. The specific design may be similar to the currently available Travenol germidicidal chamber or the previously cited Hogan patent.

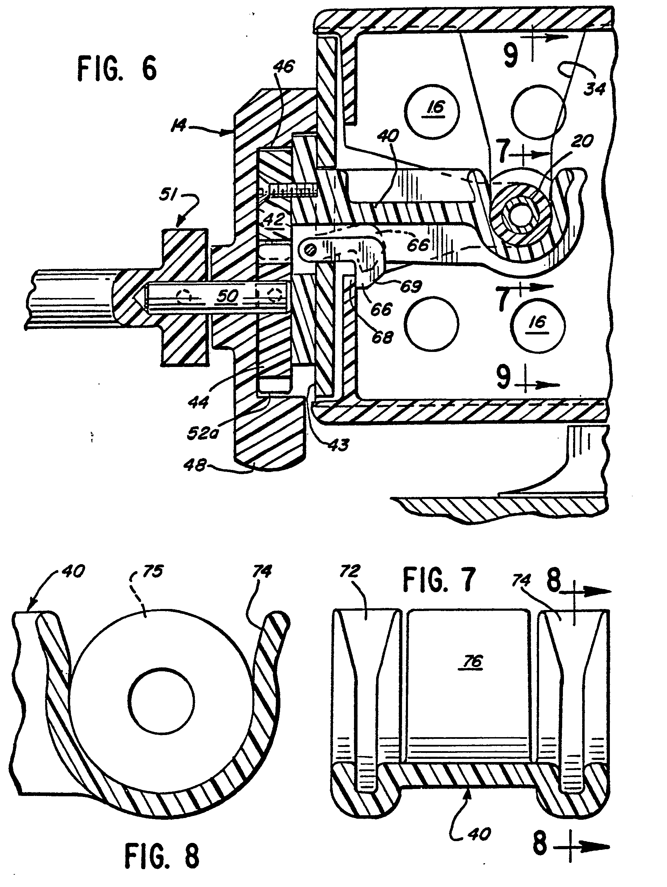

Turning particularly to Figure 7, drawer 14 is shown to carry pivotable latch hook means 66, which is positioned to latch against latch wall 68 of housing 12 as the latch engaging site (see Figures 1, 3, and 7) for engagement of pivotable latch hook 66 and latch wall 68 in the first position of second means 40, i.e., when the connectors are separated, and when the drawer means 14 is closed. As shown in Figure 7, latch hook means 66 defines an angled face 69 which causes latch hook 66 to deflect upwardly as drawer 14 is closed when it impinges latch wall 68. As drawer 14 completes the closing action, latch hook 66 falls back again as shown in Figure 7, to prevent reopening of the drawer in that position.

However, as shown in Figures 1 and 3, the latch wall 68 only extends across a portion of the width of housing 10, with a reduced height wall 70 occupying substantially the remainder of the width of the housing. Accordingly, as second connector holding means 40 is moved by rotation of handle 51 toward the first, connected position, latch hook 66 which is carried with it is moved out of engagement with latch wall 68, being capable of passing over the top of reduced height wall 70. Thus drawer 14 can be opened after closing, when handle 51 is rotated as shown in Figure 2 to move second means 40 into the first, connected position.

Other designs of latch means and latch engaging site may also be used.

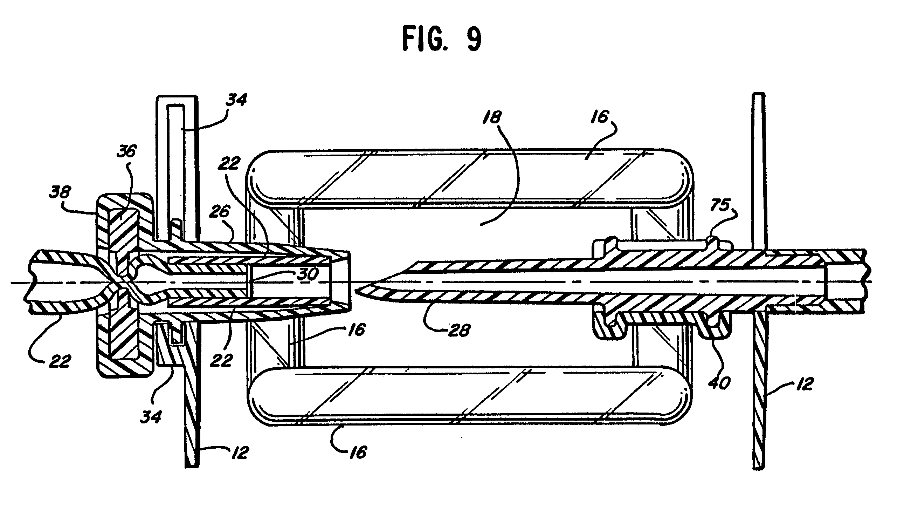

Referring to Figures 8 and 9, it can be seen that second connector holding means 40 defines a pair of slots 72, 74 for receiving corresponding flanges 751 of a connector 20 as shown in Figures 1 and 3. A separate partially cylindrical portion 76 is also provided, defining spring arm means for releasably retaining connector 20. Thus, connector 20 can snap into and out of retained relationship with second means 40.

For use of the apparatus of this invention, a patient on CAPD has typically drained spent dialysis solution from his peritoneal cavity through tubing 21 and connector 20 into a bag 24. He then inserts connector 20 into first and second holding means 34, 40 with drawer 14 and handle 51 in the position of Figure 3. Handle 51 is then rotated to the Figure 1 position, separating connector 20 from connector tubing 22. Bag 24 and connector tubing 22 are then replaced with a fresh bag 24 and tubing 22, and drawer 14 is closed to begin irradiation. Line 21 may be closed off with a slide clamp or the like in conventional manner. The molded structure comprising sleeve 26 is placed onto port 22 of the fresh bag of dialysis solution 24 to seat it in the first connector holding means 34 with slide clmap 36 being closed.

Ultraviolet lamp system 16 is activated by the closing of the drawer in a manner which may be similar to the electronic system of the Travenol germicidal chamber, with the ultraviolet irradiation continuing until the desired level of irradiation is achieved. This can be signaled to the patient by known circuitry, and an indicator light or buzzer, following which the patient rotates handle 51 again to the configuration shown in Figure 2, causing the respective connectors to connect into flow relationship with each other. Thereafter, the patient opens drawer 14 again as in Figure 3, and removes the newly connected system. He then opens clamp 36 and the clamp on tubing 21 to cause the fresh peritoneal dialysis solution of flow from bag 24 through tubing 21 into the patient's peritoneal cavity.

As the result of this, even a debilitated patient can make connections with ease, and with improved reliability of aseptic conditions, with the ultraviolet light irradiation greatly reducing the risk of contamination and consequent peritonitis. The respective parts exposed to ultraviolet light such as spike 20 and sleeve 26, may be made of known ultraviolet transparent materials so that their interiors as well as their exteriors may be irradiated for antibacterial effect.

## Claims

1. Radiation sterilization apparatus for a pair of connectors (20, 22) to be joined together, the connectors being carried at the ends of flow conduits, and said apparatus comprising a housing (12), means (16) within said housing for providing antibacterial radiation, and first and second means (34, 40) for holding the connectors within the housing in a position to be irradiated by said radiation, characterised by drawer means (14), carrying said first and second means (34, 40) to permit exposure of said connectors to antibacterial radiation when said drawer means is closed and to permit manual access to said connectors when mounted in said first and second means when the drawer means is opened, at least one of said first and second means being movable between first and second positions by control means (42, 44, 51) operable from outside said housing, to cause connectors carried in the first and second means to be separated from each other in the first position, and to cause said carried connectors to be joined together in the second position.

2. The apparatus of Claim 1 in which said means (16) for providing radiation is a source of ultraviolet radiation.

3. Apparatus according to Claim 1 or 2, wherein the control means includes rack and gear means (42, 44) for relatively moving said first and second means between said first and second positions, the gear (44) of the rack and gear means having gear teeth (52) of increasing pitch from its centre of rotation about at least a portion of its periphery, the rack (42) of the rack and gear means having mating teeth (56) correspondingly spaced along its length in positions to permit relatively linear motion between the rack and gear as the gear rolls along the rack, whereby the mechanical advantage of the rack and gear means varies with the position of the gear along the rack.

4. The apparatus of Claim 3 in which said mechanical advantage is substantially at its maximum when the rack and gear means are at the second position.

5. The apparatus of Claim 3 or 4 in which the teeth (56) of said rack are positioned at differing distances from an axis of relative motion of the first and second means (34, 40), between the first and second positions, said differing distances inversely corresponding to the differing pitches of the gear teeth (52) to permit relatively linear motion along said axis.

6. The apparatus of Claim 5 in which the sum of the pitch of each gear tooth (52) and the distance from said axis of a rack tooth (56) meshing against said gear tooth is substantially constant from gear tooth to gear tooth.

7. The apparatus of any preceding claim in which one of said housing (12) and second means (40) has latch means (66), said second means being movable by the control means (42, 44) and the other of said housing and second means having a latch engaging site (68) positioned to engage said latch means in the first position and to be spaced from the latch means in the second position, whereby said drawer means (14) cannot be reopened after closing until said second means is moved to the second position.

8. The apparatus of any preceding claim in which one of said first and second means (34, 40) defines spring arm means for releasably retaining a connector.

9. The apparatus of any one of Claims 3 to 8 in which said gear (44) is attached to a rotatable shaft (50), whereby rotation of such shaft and gear causes the rack (42) to move in a direction transverse of the shaft, said shaft terminating in a rotatable handle (51).

10. The apparatus of Claim 9 in which said shaft carries rotatable handle means adapted to rotate substantially 180° to cause said second means to move between the first and second positions.

11. Radiation sterilization apparatus according to any preceding claim, in combination with a pair of connectors (20, 22), the connectors being held by the first and second means, one of said connectors being a port (22) of a peritoneal dialysis solution bag (24) and the other of said connectors (20) being attached to tubing (21) of a peritoneal transfer set.

## Patentansprüche

1. Strahlensterilisationvorrichtung für zwei zusammenzufürgende Verbinder (20, 22), die an den Enden von Strömungsleitungen angeordnet sind, wobei die Vorrichtung umfaßt: ein Gehäuse (12), eine Einrichtung (16) in dem Gehäuse, die antibakterielle Strahlung liefert, und eine erste und zweite Einrichtung (34, 40), die die Verbinder im Gehäuse in einer von der Strahlung zu bestrahlenden Stellung hält, gekennzeichnet durch eine Schublade (14), die die erste und zweite Einrichtung (34, 40) trägt und ein Bestrahlen der Verbinder mit antibakterieller Strahlung gestattet, wenn die Schublade geschlossen ist, und manuellen Zugriff zu den in der ersten und zweiten Einrichtung befestigten Verbindern gestattet, wenn die Schublade geöffnet ist, wobei wenigstens entweder die erste oder die zweite Einrichtung durch eine von außerhalb des Gehäuses betätigbare Betätigungseinrichtung (42, 44, 51) zwischen einer ersten und einer zweiten Stellung bewegbar ist, so daß in der ersten Stellung eine Trennung von von der ersten und zweiten Einrichtung gehaltenden Verbindern voneinander und in der zweiten Stellung ein Zusammenfügen der gehaltenen Verbinder bewirkt wird.

2. Vorrichtung nach Anspruch 1, wobei die Strahlung liefernde Einrichtung (16) eine UV-Strahlungsquelle ist.

3. Vorrichtung nach Ansruch 1 oder 2, wobei die Betätigungseinrichtung einen Zahnstrangenmechanismus (42, 44) aufweist, der die erste und zweite Einrichtung relativ zueinander zwischen der ersten und zweiten Stellung bewegt, wobei das Zahnrad (44) des Zahnstangenmechansimus ein Zahnsegment (52) mit von Rotationsmittelpunkt um wenigstens einen Teil seines Umfangs zunehmender Teilung aufweist, wobei die Zahnstange (42) des Zahnstangenmechanismus Gegenzähne (56) aufweist, die über die Zahnstangenlänge in Stellungen entsprechend beabstandet sind, die bei Ablaufen des Zahnrads entlang der Zahnstange eine relative Linearbewegung zwischen Zahnstange und Zahnrad gestatten, wodurch die mechanische Kraftverstärkung des Zahnstangenmechanismus sich mit der Stellung des Zahnrads entlang der Zahnstange ändert.

4. Vorrichtung nach Anspruch 3, wobei die mechanische Kraftverstärkung im wesentlichen ihr Maximum hat, wenn sich der Zahnstangemechanismus in der zweiten Stellung befindet.

5. Vorrichtung nach Anspruch 2 oder 4, wobei Zähne (56) der Zahnstange von einer Relativbewegungsachse der ersten und zweiten Einrichtung (34, 40) zwischen der ersten und zweiten Stellung unterschiedlich beabstandet sind, wobei die unterschiedlichen Abstände den unterschiedlichen Teilungen des Zahnsegments (52) umgekehrt entsprechen und dadurch eine relative Linearbewegung entlang der Achse gestatten.

6. Vorrichtung nach Anspruch 5, wobei die Summe der Teilung jedes Zahnradzahns (52) und der Abstand von der Achse eines mit dem Zahnradzahn kämmenden Zahns (56) der Zahnstange von einem Zahnradzahn zum anderen im wesentlichen konstant ist.

7. Vorrichtung nach einem der vorhergehenden Ansprüche, wobei entweder das Gehäuse (12) oder die zweite Einrichtung (40) einen Riegel (66) aufweist, die zweite Einrichtung von der Betätigungseinrichtung (42, 44) bewegbar ist und das jeweils andere der Elemente Gehäuse bzw. zweite Einrichtung eine Riegeleinriffsstelle (68) aufweist, die so postioniert ist, daß sie in der ersten Stellung mit dem Riegel in Eingriff steht und in der zweiten Stellung vom Riegel beabstandet ist, wodurch die Schublade (14) nach dem Schließen erst wieder geöffnet werden kann, wenn die zweite Einrichtung in die zweite Stellung bewegt ist.

8. Vorrichtung nach einem der vorhergehenden Ansprüche, wobei entweder die erste oder die zweite Einrichtung (34, 40) einen Federschenkel zum lösbaren, Festlegen eines Verbinders definiert.

9. Vorrichtung nach einem der Ansprüche 3 bis 8, wobei das Zahnrad (44) an einer Welle (50) befestigt ist, wodurch eine Drehung der Welle und des Zahnrads eine Bewegung der Zahnstange (42) in Querrichtung zur Welle bewirkt, wobei die Welle in einem drehbaren Griff (51) endet.

10. Vorrichtung nach Anspurch 9, wobei die Welle ein drehbares Griffelement trägt, das im wesentlichen um 180° drehbar ist und eine Bewegung der zweiten Einrichtung zwischen der ersten und zweiten Stellung bewirkt.

11. Strahalensterilisationsvorichtung nach einem der vorhergehenden Ansprüche in Kombination mit zwei Verbindern (20, 22), wobei die Verbinder von der ersten und zweiten Einrichtung gehalten sind und wobei einer der Verbinder ein Anschlußkanal (22) eines Peritonealdialyselösungs-Beutels (24) ist und der andere Verbinder (20) an einen Schlauch (21) eines Peritoneal-Transfersets angeschlossen ist.

**Revendications**

1. Appareil de stérilisation par radiation pour une paire de connecteurs (20, 22) devant être unis, les connecteurs étant montés aux extrémités de conduits pour flux, et ledit appareil comprenant une enveloppe (12), à l'intérieur de ladite enveloppe, des moyens (16) pour produire une radiation bactéricide, et un premier et un deuxième moyens (34, 40) pour maintenir les connecteurs à l'intérieur de l'enveloppe dans une position permettant une irradiation par ladite radiation, caractérisé par des moyens de glissière (14) portant lesdits premier et deuxième moyens (34, 40) pour permettre l'exposition desdits connecteurs à la radiation bactéricide lorsque ledit moyen de glissière est fermé et pour permettre un accès manuel auxdits connecteurs alors montés dans le premier et le deuxième moyens lorsque le moyen de glissière est ouvert, l'un au moins desdits premier et deuxième moyens étant mobile entre une première et une deuxième positions par des moyens de commande (42, 44, 51) manoeuvrables de l'extérieur de ladite enveloppe, pour faire que les connecteurs portés dans les premier et deuxième moyens se séparent l'un de l'autre dans la première position et pour faire que lesdits connecteurs portés soient réunis dans la deuxième position.

2. Appareil selon la revendication 1, caractérisé par le fait que lesdits moyens (16) d'irradiation sont une source de radiation ultraviolette.

3. Appareil selon la revendication 1 ou la revendication 2, caractérisé par le fait que le moyen de contrôle comprend des moyens d'engrenage à crémaillère (42, 44) pour le déplacement relatif desdits premier et deuxième moyens entre lesdites première et deuxième positions, l'engrenage (44) du moyen d'engrenage à crémaillère ayant des dents d'engrenage (52) de pas croissant depuis son centre de rotation sur au moins une partie de sa périphérie, la crémaillère (42) du moyens d'engrenage à crémaillère ayant des dents appariées (56) espacées de façon correspondante sur sa longueur pour permettre un mouvement relativement linéaire entre la crémaillère et l'engrenage à mesure que l'engrenage roule sur la crémaillère, l'effet mécanique du moyen d'engrengage à crémaillère variant avec la position de l'engrenage sur la crémaillère.

4. Appareil selon la revendication 3, caractérisé par le fait que ledit effet mécanique est sensiblement maximal lorsque les moyens d'engrenage à crémaillère sont dans la deuxième position.

5. Appareil selon la revendication 3 ou la revendication 4, caractérisé par le fait que les dents (56) de ladite crémaillère sont positionnées à des distances différentes à partir d'un axe de déplacement relatif des premiuer et deuxième moyens (34, 40) entre les première et deuxième positions, lesdites distances différentes étant en relation inverse aux différents par des dents d'engrenage (52) pour permettre un mouvement relativement linéaire le long dudit axe.

6. Appareil selong la revendication 5 caractérisé par le fait que la somme du pas de chacune des dents d'engrenage (52) et de la distance à partir dudit axe d'une dent de crémaillère (56) s'engrenant contre ladites den d'engrenage est sensiblement constante d'une dent d'engregange à une autre.

7. Appareil selon l'une quelconque des revendications précédentes, caractérisé par le fait que l'un desdits enveloppe (12) et deuxième moyen (40) possède des moyens de loquet (66), ledit deuxième moyen étant mobile par le moyen de controîle (42, 44), et l'autre desdits enveloppe et deuxième moyen ayant un emplacement d'engagement de loquet (68) positionné pour engager ledit moyen de loquet dans la première position et pour être à distance du moyen de loquet dans la deuxième position, ledit moyen de glissière (14) ne pouvant être réouvert après fermeture avant que ledit deuxième moyen ne soit venu dans la deuxième position.

8. Appareil selon l'une quelconque des revendications précédentes dans laquel l'un desdits premier et deuxième moyens (38, 40) définit un moyen de bras de ressort pouvant maintenir un connecteur de façon détachable.

9. Appareil selon l'une quelconque des revendications 3 à 8, caractérisé par le fait que ledit engrenage (44) est fixé sur un arbre tournant (50), la rotation de cet arbre et de l'engrenage faisant se déplacer la crémaillère (42) dans le sens transversal à l'arbre, ledit arbre se terminant par une manette pouvant tourner (51).

10. Appareil selon la revendication 9 caractérisé par la fait que ledit arbre porte des moyens de manette pivotante adaptée pour tourner sur sensiblement 180° pour faire que ledit deuxième moyen se déplace entre une première et une deuxième positions.

11. Appareil de stérilisation par radiation selon l'une quelconque des revendications précédentes, associé à une paire de connecteurs (20, 22), les connecteurs étant portés par le premier et le deuxième moyens, l'un desdits connecteurs étant un orifice (22) d'un sac de solution de dialyse péritonéale (24) et l'autre desdits connecteurs (20) étant fixé sur le tube (21) d'une unité de transfert péritonéal.

FIG. 1

FIG. 2

FIG. 3

FIG. 4

FIG. 5

2

FIG. 6

FIG. 7

FIG. 8

# FIG. 9